# EUROPÄISCHE PATENTSCHRIFT

(11) **EP 3 102 146 B1**
(45) Veröffentlichungstag und Bekanntmachung des Hinweises auf die Patenterteilung: **10.04.2019**
(21) Anmeldenummer: 15707291.9
(22) Anmeldetag: 04.02.2015
(51) Int. Cl.: A61C 13/34, G06F 17/50, G06T 19/20, A61C 13/00

(54) **VERFAHREN ZUR COMPUTERGESTÜTZTEN BEARBEITUNG EINES DIGITALEN 3D-MODELLS**
METHOD FOR THE COMPUTER-AIDED EDITING OF A DIGITAL 3D MODEL
PROCÉDÉ DE TRAITEMENT ASSISTÉ PAR ORDINATEUR D'UN MODÈLE 3D NUMÉRIQUE

(30) Priorität: 04.02.2014 DE 102014201993
(43) Veröffentlichungstag der Anmeldung: 14.12.2016
(73) Patentinhaber: Sirona Dental Systems GmbH, 64625 Bensheim (DE)
(72) Erfinder: JORDAN, Thorsten, 64319 Pfungstadt (DE)
(74) Vertreter: Sommer, Peter
(86) Internationale Anmeldenummer: PCT/EP2015/052230
(87) Internationale Veröffentlichungsnummer: WO 2015/117973

(56) Entgegenhaltungen:
- US-A1- 2006 155 404
- US-A1- 2007 203 599
- US-A1- 2012 070 803
- US-A1- 2013 325 415

## Beschreibung

### Technisches Gebiet

Die Erfindung betrifft ein Verfahren zur computergestützten Bearbeitung eines digitalen 3D-Modells eines dentalen Objekts mittels digitaler Tools. Hierfür wird mindestens ein digitales Tool ausgewählt und eine Wirkung des Tools für das gesamte 3D-Modell berechnet.

### Stand der Technik

Bei der computergestützten Bearbeitung von 3D-Modellen, insbesondere von Zähnen, werden Tools angewandt, die die Form des 3D-Modells verändern. Die Tools wirken beispielsweise immer auf das gesamte 3D-Modell oder nur auf von Hand mittels eines Eingabemittels auswählbare Bereiche des 3D-Modells, beispielsweise auf zahntechnisch sinnvolle Stellen.

Dabei ist die genaue Wirkung des Tools für den Anwender nicht genau vorhersehbar, so dass häufig erst nach Anwendung des Tools beurteilt werden kann, ob das Ergebnis brauchbar ist. Ist das Ergebnis nicht brauchbar, so müssen die Veränderungen wieder rückgängig gemacht oder durch die Anwendung anderer Tools korrigiert werden.

Eine solche Bearbeitung erfordert einige Erfahrung hinsichtlich der Wirkung der Tools, um möglichst schnell möglichst brauchbare Ergebnisse zu erhalten. Auch die zahntechnisch sinnvolle Auswahl von Bereichen bzw. die Einschränkung der Wirkung des Tools von Hand erfordert Erfahrung auf Seiten des Anwenders.

Die Aufgabe der vorliegenden Erfindung ist es, ein Verfahren bereitzustellen, welches die Berücksichtigung zahntechnischer Kriterien sicherstellt und es auch einem unerfahrenen Anwender ermöglicht, die Wirkung eines Tools auf ein 3D-Modell eines dentalen Objekts abzuschätzen.

Die Dokumente US2006/0155404 und US2012/0070803 offenbaren auch computergestützte Bearbeitung von 3D-Modellen.

### Offenbarung der Erfindung

Die Erfindung ist wie in Anspruch 1 definiert.

Diese Aufgabe wird durch ein Verfahren zur computergestützten Bearbeitung eines digitalen 3D-Modells eines dentalen Objekts mittels digitaler Tools gelöst, wobei mindestens ein digitales Tool ausgewählt und eine Wirkung des Tools für das gesamte 3D-Modell berechnet wird. Dabei werden unterschiedliche Regionen des 3D-Modells erkannt, wobei mindestens eine Region zumindest teilweise einer okklusalen oder inzisalen oder labialen oder bukkalen oder distalen oder mesialen oder lingualen oder palatinalen Fläche des dentalen Objekts entspricht und wobei das Tool auf die unterschiedlichen Regionen unterschiedlich wirkt. Die berechnete Wirkung für das gesamte 3D-Modell wird als Vorschlagsmodell bereitgestellt und zusammen mit dem unveränderten 3D-Modell angezeigt. Anschließend wird das Vorschlagsmodell verworfen oder teilweise oder vollständig angenommen. Bei vollständiger Annahme wird das Vorschlagsmodell als ein Ergebnismodell angezeigt. Bei teilweiser Annahme wird das 3D-Modell insgesamt oder mindestens eine Region oder mindestens ein Teilbereich des 3D-Modells als Bereich ausgewählt. Aus dem 3D-Modell und dem Vorschlagsmodell wird ein Ergebnismodell gebildet, indem ausgehend vom 3D-Modell der mindestens eine ausgewählte Bereich oder zumindest ein zentraler Teil des ausgewählten Bereichs durch einen entsprechenden Bereich des Vorschlagsmodells ersetzt oder diesem unter Anwendung mindestens eines Stärkefaktors angenähert wird, wobei das Ergebnismodell angezeigt wird.

Dieses Verfahren stellt sicher, dass sich die Wirkung eines Tool auf zahntechnisch sinnvolle Stellen des 3D-Moddels beschränkt und ermöglicht es weiterhin einem Anwender, diese mögliche Wirkung des Tools im Vergleich zum 3D-Modell zu betrachten, das Tool auch nur teilweise wirken zu lassen und jeder Zeit ganz oder teilweise zum ursprünglichen 3D-Modell zurückzukehren.

Ein dentales Objekt, also ein oder mehrere Zähne bzw. eine Zahnrestauration, weist verschiedene Regionen bzw. Flächen auf. Dies sind eine Unterseite bzw. ein Zahnwurzelbereich sowie in fünf verschiedene Richtungen zeigende Oberflächenregionen, deren dentalspezifische Bezeichnung sich nach der Ausrichtung und Position des dentalen Objekts im Kiefer richtet. Ein Backenzahn weist beispielsweise eine Kaufläche, also eine Okklusalfläche, sowie seitliche Flächen, mesial, distal, bukkal und lingual oder palatinal auf. Die Okklusalfläche weist weiterhin Einkerbungen bzw. Fissuren sowie Höcker auf. Ein Schneidezahn weist eine inzisale Fläche oder Kante als Kaufläche sowie ebenfalls seitliche Flächen auf, die als mesiale, distale, labiale und lingual oder palatinal bezeichnet werden.

Mindestens eine zahnspezifische Region wird erfindungsgemäß erkannt. Das Erkennen dieser unterschiedlichen dentaltypischen Regionen bzw. zumindest einer dieser Regionen ermöglicht das Variieren der Wirkung des Tools gemäß zahntechnischer Kriterien, beispielsweise das Beschränken auf bestimmte Regionen und/oder das Verstärken zur Mitte einer Region hin.

Es kann beispielsweise sichergestellt werden, dass ein Tool zum Anschmiegen an einen Nachbarzahn im Wesentlichen auf die mesiale und/oder distale Fläche oder sogar insbesondere verstärkt auf einen zentralen Bereich dieser Fläche wirkt und die angrenzenden Regionen, beispielsweise die linguale Fläche oder die Inzisal- oder Okklusalfläche lediglich an die Änderungen der mesialen und/oder distalen Fläche angeglichen werden, soweit dies nötig ist. Die Wirkung eines Tools zur Herstellung von Kontaktstellen zu einem gegenüberliegenden Zahn kann hingegen beispielsweise auf die Inzisal- oder Okklusalfläche oder sogar auf die Höcker der Okklusalfläche beschränkt bleiben.

Beispielsweise mittels eines geeigneten Eingabemittels kann der Anwender weiterhin im Rahmen der zahntechnisch sinnvollen und daher vorgeschlagenen Wirkung des Tools einen oder auch mehrere Bereiche des 3D-Modells auswählen, für welche die Wirkung des Tools übernommen werden soll und dadurch das 3D-Modell an individuelle Gegebenheiten anpassen. Diese ausgewählten Bereiche können zueinander beabstandet sein, aneinander angrenzen oder sich überlappen. Die Bereiche können vollständig oder teilweise mit den erkannten Regionen übereinstimmen. Die teilweise Annahme des Vorschlags ermöglicht es dann, das Tool auf einen oder mehrere ausgewählte Bereiche anzuwenden. Weiterhin ermöglicht das teilweise Annehmen nicht nur eine örtliche Einschränkung der vorgeschlagenen Wirkung des Tools sondern auch eine Einschränkung bzw. Beeinflussung hinsichtlich der vorgeschlagenen Stärke der Wirkung. So kann mittels eines Stärkefaktors die Wirkung abgeschwächt oder verstärkt werden. Der Stärkefaktor kann beispielsweise in Prozent oder Bruchteilen vom Anwender angegeben bzw. ausgewählt werden, wobei für verschiedene Teilbereiche verschiedene Stärkefaktoren wählbar sind.

So können Teilwirkungen sowohl hinsichtlich der örtlichen Ausdehnung als auch hinsichtlich der Stärke auf einfache Weise erwirkt und auch akkumuliert oder einzeln wieder rückgängig gemacht werden.

Vorzugsweise wird um den ausgewählten Bereich ein Übergangsbereich festgelegt, wobei in dem Übergangsbereich das 3D-Modell stufenweise an das durch den Stärkefaktor gewichtete Vorschlagsmodell angeglichen wird.

Durch das Festlegen des Übergangsbereichs wird beispielsweise ein sanfter und/oder glatter Übergang zwischen gegenüber dem 3D-Modell veränderten und unveränderten Bereichen ermöglicht. Die Stufen des Übergangs können beliebig klein und/oder im Rahmen der Auflösung des 3D-Modells gewählt werden, um einen möglichst glatten Übergang zu erreichen. Die Stufen können alle gleich oder auch gemäß einer anderen Funktion über den Übergangsbereich verteilt unterschiedlich sein, so dass der Übergangsbereich beispielsweise einen linearen, exponentiellen, parabelförmigen oder einen beliebigen anderen Verlauf aufweist. Der Übergangsbereich und/oder die Art der Stufen können automatisch durch eine Recheneinheit oder auch vom Anwender bestimmt werden.

Vorzugsweise wird das Vorschlagsmodell in der gemeinsamen Darstellung transparent oder halbtransparent angezeigt.

So kann der Anwender sowohl das 3D-Modell als auch den Vorschlag gleichzeitig wahrnehmen und die Wirkung des Tools besonders gut abschätzen.

Vorzugsweise werden Bereiche, in denen das Vorschlagsmodell vom 3D-Modell in der gemeinsamen Darstellung überdeckt wird, automatisch erkannt und auf dem 3D-Modell angezeigt.

Wird das 3D-Modell opak dargestellt, so kann es sein, dass das Vorschlagsmodell vollständig oder zumindest stellenweise durch das 3D-Modell verdeckt wird. Das Darstellen dieser Bereiche auf dem 3D-Modell, beispielsweise das farbliche Markieren, ermöglicht es einem Anwender diese Bereiche trotzdem wahrzunehmen bzw. die Wirkung des Tools auch in diesem Bereich abzuschätzen.

Vorzugsweise wird ein Abstand zwischen dem Vorschlagsmodell und dem 3D-Modell mittels einer Farbkodierung des Vorschlagsmodells angezeigt.

Eine Farbkodierung des Abstands vereinfacht es die Wirkung des Tools abzuschätzen.

Vorzugsweise werden das Ergebnismodell und das 3D-Modell abwechselnd angezeigt.

Hierdurch wird es dem Anwender ermöglicht, die von ihm ausgewählte Wirkung des Tools mit dem ursprünglichen 3D-Modell zu vergleichen und zu überprüfen.

Vorzugsweise wird der mindestens eine ausgewählte Bereich mittels eines Eingabegeräts ausgewählt.

Ein Eingabegerät ermöglicht ein einfaches Auswahlverfahren. Beispielsweise kann mit einer Computermaus oder ähnlichem und einem dargestellten und durch die Computermaus bewegbaren Zeiger ein Bereich ausgewählt und/oder verschoben und/oder verformt werden. Oder es kann mittels einer Computermaus oder ähnlichem ein beispielsweise punkt- oder kreisförmiger Zeiger zum Auswählen, wie ein Marker oder Malstift, über die gewünschten Bereich bewegt werden.

Vorzugsweise wird für das Auswählen eines Bereichs eine Markierung auf dem angezeigten 3D-Modell angezeigt, die mittels eines Eingabemittels bewegbar und auswählbar ist.

Das Verändern oder Bewegen einer Markierung auf dem 3D-Modell ermöglicht ein einfaches Auswählen von Teilbereichen.

Vorzugsweise wird ein dem von der Markierung erfassten ausgewählten Bereich entsprechender Bereich des Vorschlagsmodells opak angezeigt und der ausgewählte Bereich des 3D-Modells nicht oder transparent angezeigt.

So lässt sich die Wirkung des Tools im ausgewählten Bereich bzw. die Wirkung der Auswahl direkt und einfach erkennen. Dies ermöglicht eine einfache und schnelle Kontrolle.

Vorzugsweise wird mittels eines Eingabegeräts mindestens eine Linie auf dem angezeigten 3D-Modell definiert und/oder verändert.

Mittels einer oder mehrerer Linien, können die Grenzen von auszuwählenden Bereichen markiert werden. Die Linien können beispielsweise frei Hand mittels des Eingabegeräts auf dem 3D-Modell gezogen werden. Oder es können gerade oder auch gebogene Linien mittels des Eingabegeräts ausgewählt, positioniert, kombiniert und/oder verändert werden.

Vorzugsweise wird mindestens ein charakteristischer Teilbereich des 3D-Modells automatisch erkannt und angezeigt und ist mittels eines Eingabemittels veränderbar und/oder auswählbar.

Manche Objekte und damit auch die dazugehörigen 3D-Modelle haben charakteristische Bereiche. Zähne zum Beispiel haben ganz typische Formen, wie beispielsweise die Höcker der Backenzähne. Das automatische Erkennen solcher charakteristischen Strukturen vereinfacht die Bearbeitung des 3D-Modells.

Vorteilhafterweise werden die unterschiedlichen Regionen anhand von gemittelten Normalenvektoren erkannt.

Da die Form eines Zahns sich zumindest näherungsweise aus mehreren zumindest im Wesentlichen geraden Flächen zusammensetzt, kann der Normalenvektor zur Erkennung von Regionen mit unterschiedlicher Ausrichtung verwendet werden.

Hierfür kann beispielsweise für jeden Punkt des 3D-Modells ein Normalenvektor, also eine gemittelte senkrechte Richtung, bestimmt werden. Anhand der Normalenvektoren können dann entweder möglichst große Bereiche von Punkten mit einem ähnlich ausgerichteten Normalenvektor zusammengefasst werden und/oder jeder Punkt kann entsprechend der Ausrichtung seines Normalenvektors einer der zu erwartenden Richtungen, wie bukkal, mesial, okklusal, distal etc. und damit der entsprechenden Gruppe bzw. Fläche zugeordnet werden.

Vorteilhafterweise werden innerhalb einer okklusalen Fläche Höcker und/oder Fissuren als charakteristische Teilbereiche automatisch erkannt.

Höcker und Fissuren stellen typische und sehr charakteristische Strukturen von Okklusalflächen dar. Gerade bei der Anwendung von Tools auf Okklusalflächen kann es wünschenswert sein, nicht die ganze Fläche gleichförmig zu verändern, sondern beispielsweise lediglich auf die Höcker einzuwirken und die Fissuren beispielsweise möglichst unverändert beizubehalten.

Vorteilhafterweise werden die Höcker und/oder Fissuren anhand der Krümmung der okklusalen Fläche erkannt.

Die Krümmung ist charakteristisch für diese Strukturen und daher ein einfaches Mittel, um Höcker und Fissuren zu erkennen. Eine Fissur weist beispielsweise eine negative Krümmung auf, während ein Höcker eine positive Krümmung hat. Zur Erkennung einer Fissur können daher beispielsweise möglichst große, zusammenhängende Bereiche von Punkten mit negativer Krümmung erkannt und zusammengefasst werden. Vorteilhafterweise wirkt das Tool (T1, T2, T3) auf erkannte Höcker und Fissuren unterschiedlich.

Hierdurch kann sichergestellt werden, dass charakteristische Formen des dentalen Objekts, beispielsweise die Fissuren, möglichst erhalten bleiben.

Vorteilhafterweise berücksichtigt das Tool (T1, T2, T3) Nachbarzähne oder benachbarten Restaurationen und/oder gegenüberliegende Zähne oder Restaurationen und/oder ein Emergenzprofil aus mindestens einem 3D-Datensatz.

Für die Bearbeitung von dentalen Objekten ist insbesondere die Berücksichtung der umgebenden Strukturen wichtig. Diese können beispielsweise in Form von weiteren 3D-Datensätzen vorliegen. Tools können so eine Annäherung bzw. ein Anschmiegen an benachbarte Strukturen, z.B. Nachbarzähne, oder Kontaktflächen mit Gegenzähnen oder auch ein Angleichen der Größe bewirken. Auch kann beispielsweise ein gewünschtes Emergenzprofil für das dentale Objekt vorliegen und das 3D-Modell an dieses angeglichen werden. Auch ein Angleichen an eine Emergenzlinie benachbarter und/oder gegenüberliegender Zähne ist möglich.

### Kurze Beschreibung der Zeichnungen

Das erfindungsgemäße Verfahren wird anhand der Zeichnung erläutert. Es zeigen:
- Fig. 1: eine gemeinsame Darstellung eines 3D-Modells und eines Vorschlagsmodells,
- Fig. 2: die Verwendung eines Auswahltools,
- Fig. 3: die automatische Auswahl charakteristischer Teilbereiche,
- Fig. 4: ein Ergebnismodell in einer gemeinsamen Darstellung mit dem Vorschlagsmodell,
- Fig. 5: ein 3D-Modell mit automatisch erkannten Regionen. Ausführungsformen der Erfindung

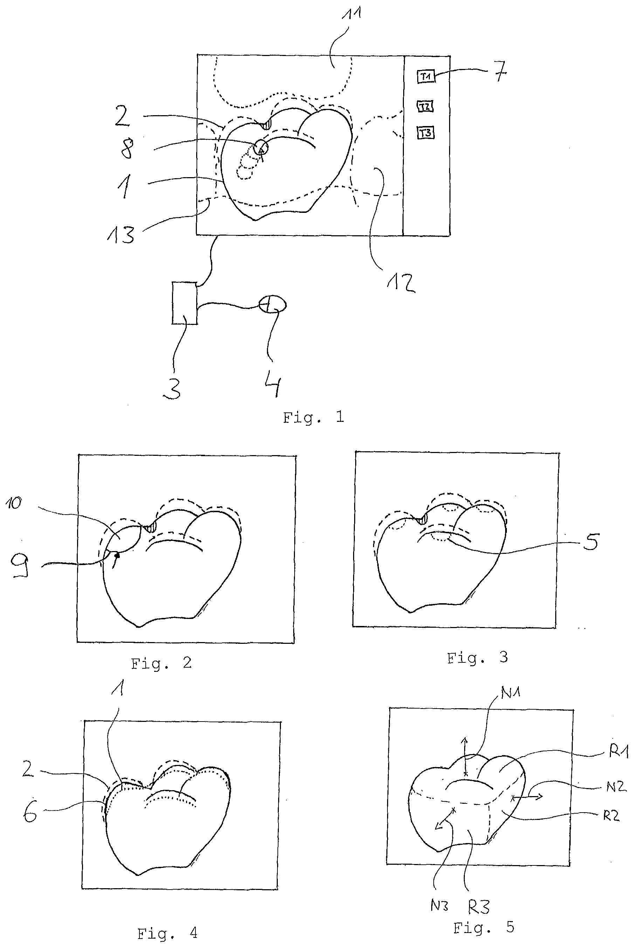

Fig. 1 zeigt eine gemeinsame Darstellung eines 3D-Modells 1 eines Backenzahns und eines Vorschlagsmodells 2. Das Vorschlagsmodell 2 wird beispielsweise mittels einer Recheneinheit 3 aus dem 3D-Modell 1 errechnet, wozu vom Anwender ein auf das 3D-Modell 1 anzuwendendes Tool T1, T2, T3 ausgewählt wird. Die Auswahl des Tools T1, T2, T3 kann beispielsweise mittels angezeigter Auswahlbuttons 7 und eines Eingabemittels 4, beispielsweise einer Computermaus, vorgenommen werden. Das Tool T1, T2, T3 wirkt beispielsweise pixelweise, so dass jedem Pixel des 3D-Modells 1 ein Pixel des Vorschlagsmodells 2 entspricht.

Ein Tool T1, T2, T3 kann beispielsweise eine Vergrößerung des 3D-Modells oder ein Anschmiegen an einen Nachbarzahn bewirken. Es können auch Tools T1, T2, T3 zur Herstellung von Kontaktstellen zu gegenüberliegenden Zähnen oder zur Anpassung des Verlauf seitlicher Flächen eines Zahns zur Veränderung und/oder Anpassung des Gingivaverlaufs, also zur Erreichung eines gewünschten Emergenzprofils, vorgesehen sein. Diese Tools T1, T2, T3 können hierfür beispielsweise zusätzlich auf 3D-Datensätze eines oder mehrerer gegenüberliegender Zähne oder Restaurationen 11, eines oder beider benachbarter Zähne oder Restaurationen 12 oder eines Emergenzprofils 13 zurückgreifen.

Um sowohl das 3D-Modell 1 als auch das Vorschlagsmodell 2 gut erkennen zu können, kann beispielsweise das 3D-Modell 1 opak und das Vorschlagsmodell 2 transparent oder teiltransparent dargestellt werden. Um das Vorschlagsmodell 2 besser einschätzen zu können, kann beispielsweise der Abstand des Vorschlagsmodells 2 zum 3D-Modell 1 durch eine Farbkodierung des Vorschlagsmodells 2 dargestellt werden.

Wird das Vorschlagsmodell 2 in der gemeinsamen Darstellung vom 3D-Modell 1 teilweise oder ganz verdeckt, so können die dem verdeckten Teil des Vorschlagsmodells 2 entsprechenden Bereiche und/oder Pixel des 3D-Modells 1 farblich markiert werden, wie dies durch den schraffierten Bereich in Fig. 1 angedeutet ist.

Es ist aber auch möglich die betroffenen Bereich des 3D-Modells 1 transparent und die entsprechenden Bereiche des Vorschlagsmodells 2 opak darzustellen. Bei einer solchen Darstellung besteht jedoch die Gefahr, dass nicht mehr klar zu erkennen ist, welche dargestellten Bereiche zu welchem Modell gehören. Dies kann jedoch beispielsweise durch eine klare farbliche Trennung behoben werden, indem das eine Modell beispielsweise in Rottönen und das andere Modell beispielsweise in Grüntönen dargestellt wird.

Das Annehmen des Vorschlagsmodells 2 oder das Annehmen von Teilen kann beispielsweise mittels des Eingabemittels 4 vorgenommen werden. Mit dem Eingabemittel 4 kann beispielsweise das gesamte 3D-Modell 1 durch anklicken oder eingeben ausgewählt werden. Soll das Tool T1, T2, T3 nur auf Teilbereiche des 3D-Modells 1 wirken, so kann dieses beispielsweise durch das Markieren von dann ausgewählten Bereichen 10 mittels einer über die Bereiche 10 bewegbaren Markierung 8, wie in Fig. 1 angedeutet, oder durch das Eingrenzen eines ausgewählten Bereichs 10 mittels Linien 9, die mittels des Eingabemittels 4 auf das 3D-Modell 1 gemalt, verschoben, verändert und/oder zusammengefügt werden, erfolgen. Das Auswählen eines Bereichs 10 mittels Linien 9 ist beispielhaft in Fig. 2 dargestellt.

Das Auswählen eines Bereichs 10 kann auch durch das Auswählen erkannter Strukturen erfolgen. Die Recheneinheit 3 kann charakteristische Teilbereiche 5, beispielsweise Höcker einer Okklusalfläche, ermitteln, die dann, wie in Fig. 3 gezeigt, beispielsweise durch eine Grenzlinie, hier gepunktet, angezeigt und mittels des Eingabemittels 4 ausgewählt und/oder verändert, beispielsweise vergrößert und als ausgewählter Bereich 10 definiert werden können. Höcker und Fissuren einer Okklualfläche eines Zahns können beispielsweise anhand der Krümmung des 3D-Modells gefunden werden. Während das 3D-Modell im Bereich der Höcker eine stärkere positive Krümmung aufweist, liegt im Bereich einer Fissur eine stärkere negative Krümmung vor.

Das Annehmen des Vorschlags kann auch hinsichtlich der Ausprägung bzw. Stärke der Wirkung ganz oder teilweise geschehen. So kann mittels eines Stärkefaktors S die Wirkung des Tools T1, T2, T3 abgeschwächt oder auch verstärkt werden, indem der 3D-Datensatz beispielsweise nicht vollständig an den Vorschlag herangeführt bzw. durch diesen ersetzt wird, sondern nur teilweise. Der Stärkefaktor S kann beispielsweise von einem Anwender auswählbar oder eingebbar sein, beispielsweise in Prozent oder Bruchteilen. Wählt der Anwender beispielsweise für einen ausgewählten Bereich 10 einen Stärkefaktor S von 50%, dann wird das 3D-Modell 1 in dem ausgewählten Bereich 10 um 50% an den Vorschlag herangeführt. Dies kann beispielsweise pixelweise geschehen, so dass für ein Ergebnismodell 6 jeder Pixel in dem ausgewählten Bereich 10 auf eine Position auf der Hälfte des Abstands der beiden Modelle gesetzt wird.

In Fig. 4 ist ein Ergebnismodell 6 beispielhaft dargestellt, welches durch die Annahme des Vorschlagsmodells 2 in zwei Teilbereichen mit jeweils unterschiedlichem Stärkefaktor S erhalten wurde. Das Ergebnismodell 5 ist mit einer durchgezogenen Linie dargestellt, während das Vorschlagsmodell 2 mit gestrichelter Linie und das 3D-Modell 1 mit gepunkteter Linie dargestellt sind. Im Bereich der beiden vorderen Höcker, einem ersten ausgewählten Bereich 10, wurde der Vorschlag mit voller Wirkung, also zu 100% angenommen. Im Bereich der beiden hinteren Höcker, einem zweiten ausgewählten Bereich 10, wurde der Vorschlag nur teilweise, nämlich zu ca. 50% angenommen. Um die beiden ausgewählten Bereiche 10 herum wurde beispielsweise automatisch ein Übergangsbereich definiert und ein glatter Übergang zwischen den gegenüber dem 3D-Modell 1 unveränderten Bereichen und den beiden ausgewählten Bereichen 10 erreicht.

Zur Überprüfung des Ergebnisses kann das Ergebnismodell 6 beispielsweise zeitlich abwechselnd mit dem 3D-Modell 1 dargestellt werden, um einen Vergleich zu ermöglichen.

Um sicher zu stellen, dass das Tool T1, T2, T3 nur in einem zahntechnisch sinnvollen Maß auf das 3D-Modell 1 einwirkt, variiert die Wirkung des Tools T1, T2, T3 für unterschiedliche Regionen R1, R2, R3 des 3D-Modells. Hierfür werden unterschiedliche Regionen R1, R2, R3 des 3D-Modells automatisch erkannt, beispielsweise eine Okklusalfläche R1, eine mesiale Fläche R2 und eine Lingualfläche R3, wie in Fig. 5 skizziert. Dies kann beispielsweise anhand eines Normalenvektors N1, N2, N3 geschehen, indem zu jedem Punkt des 3D-Modells ein Normalenvektor N1, N2, N3 bestimmt wird und Regionen mit Normalenvektoren N1, N2, N3 mit einer zumindest näherungsweise gleichen Richtung zu einer Region R1, R2, R3 zusammengefasst werden. Es kann auch jeder Punkt entsprechend der Richtung seines Normalenvektors N1, N2, N3 einer bestimmten Ausrichtung, beispielsweise okklusal, mesial, lingual etc., und damit der entsprechenden Region R1, R2, R3 zugeordnet werden.

Auch Höcker und/oder Fissuren einer Kaufläche bzw. Okklusalfläche können als Regionen R1, R2, R3 automatisch erkannt werden, so dass auch hinsichtlich dieser Strukturen die Wirkung eines Tools automatisch variiert werden kann. Eine Erkennung ist, wie vorab beschrieben, beispielsweise anhand der Krümmung des 3D-Modells im Bereich der Okklusalfläche möglich.

Je nach Tool, kann die Wirkung beispielsweise auf eine oder mehrere erkannte Regionen R1, R2, R3 oder auch auf einen zentralen Bereich der Region R1, R2, R3 beschränkt sein.

### Bezugszeichenliste

- 1: 3D-Modell
- 2: Vorschlagsmodell
- 3: Recheneinheit
- 4: Eingabemittels
- 5: charakteristischer Teilbereich
- 6: Ergebnismodell
- 7: Auswahlbutton
- 8: Markierung
- 9: Linie
- 10: ausgewählter Bereich
- 11: gegenüberliegender Zahn
- 12: Nachbarzahn
- 13: Emergenzprofil
- N1: Normalenvektor
- N2: Normalenvektor
- N3: Normalenvektor
- R1: Region
- R2: Region
- R3: Region
- S: Stärkefaktor
- T1: Tool
- T2: Tool
- T3: Tool

## Patentansprüche

1. Verfahren zur computergestützten Bearbeitung eines digitalen 3D-Modells (1) eines dentalen Objekts mittels digitaler Tools (T1, T2, T3), wobei mindestens ein digitales Tool (T1, T2, T3) ausgewählt und eine Wirkung des Tools (T1, T2, T3) für das gesamte 3D-Modell (1) berechnet wird, wobei
- unterschiedliche Regionen (R1, R2, R3) des 3D-Modells (1) erkannt werden, wobei mindestens eine Region zumindest teilweise einer okklusalen oder inzisalen oder labialen oder bukkalen oder distalen oder mesialen oder lingualen oder palatinalen Fläche des dentalen Objekts entspricht,
- das Tool (T1, T2, T3) auf unterschiedliche Regionen (R1, R2, R3) unterschiedlich wirkt,
- die berechnete Wirkung für das gesamte 3D-Modell (1) als Vorschlagsmodell (2) bereitgestellt und zusammen mit dem unveränderten 3D-Modell (1) angezeigt wird und dass
- das Vorschlagsmodell (2) verworfen oder teilweise oder vollständig angenommen wird,
- wobei bei vollständiger Annahme das Vorschlagsmodell (2) als ein Ergebnismodell (6) angezeigt wird und
- bei teilweiser Annahme das 3D-Modell (1) insgesamt oder mindestens eine Region (R1, R2, R3) oder mindestens ein Teilbereiche des 3D-Modells (1) als Bereich (10) ausgewählt wird, ein Ergebnismodell (6) aus dem 3D-Modell (1) und dem Vorschlagsmodell (2) gebildet wird, indem ausgehend vom 3D-Modell (1) der mindestens eine ausgewählte Bereich (10) oder zumindest ein zentraler Teil des ausgewählten Bereichs (10) durch einen entsprechenden Bereich des Vorschlagsmodells (2) ersetzt oder diesem unter Anwendung mindestens eines Stärkefaktors (S) angenähert wird und das Ergebnismodell (6) angezeigt wird.

2. Verfahren nach Anspruch 1, **dadurch gekennzeichnet, dass** um den ausgewählten Bereich ein Übergangsbereich festgelegt wird, wobei in dem Übergangsbereich das 3D-Modell (1) stufenweise an das durch den Stärkefaktor (S) gewichtete Vorschlagsmodell (2) angeglichen wird.

3. Verfahren nach Anspruch 1 oder 2, **dadurch gekennzeichnet, dass** das Vorschlagsmodell (2) in der gemeinsamen Darstellung transparent oder halbtransparent angezeigt wird.

4. Verfahren nach einem der Ansprüche 1 bis 3, **dadurch gekennzeichnet, dass** Bereiche, in denen das Vorschlagsmodell (2) vom 3D-Modell (1) in der gemeinsamen Darstellung überdeckt wird, automatisch erkannt und auf dem 3D-Modell (1) angezeigt werden.

5. Verfahren nach einem der Ansprüche 1 bis 4, **dadurch gekennzeichnet, dass** ein Abstand zwischen dem Vorschlagsmodell (2) und dem 3D-Modell (1) mittels einer Farbkodierung des Vorschlagsmodells (2) angezeigt wird.

6. Verfahren nach einem der Ansprüche 1 bis 5, **dadurch gekennzeichnet, dass** das Ergebnismodell (6) und das 3D-Modell (1) abwechselnd angezeigt werden.

7. Verfahren nach einem der Ansprüche 1 bis 6, **dadurch gekennzeichnet, dass** der mindestens eine ausgewählte Bereich (10) mittels eines Eingabegeräts ausgewählt wird.

8. Verfahren nach einem der Ansprüche 1 bis 7, **dadurch gekennzeichnet, dass** für das Auswählen eines Bereichs (10) eine Markierung (8) auf dem angezeigten 3D-Modell (1) angezeigt wird, die mittels eines Eingabemittels (4) bewegbar und auswählbar ist.

9. Verfahren nach Anspruch 8, **dadurch gekennzeichnet, dass** ein dem von der Markierung (8) erfassten ausgewählten Bereich (10) entsprechender Bereich des Vorschlagsmodells (2) opak angezeigt und der ausgewählte Bereich (10) des 3D-Modells (1) nicht oder transparent angezeigt wird.

10. Verfahren nach einem der Ansprüche 1 bis 9, **dadurch gekennzeichnet, dass** mittels eines Eingabemittels (4) mindestens eine Linie (9) auf dem angezeigten 3D-Modell (1) definiert und/oder verändert wird.

11. Verfahren nach einem der Ansprüche 1 bis 10, **dadurch gekennzeichnet, dass** mindestens ein charakteristischer Teilbereich (5) des 3D-Modells (1) automatisch erkannt und angezeigt wird und mittels eines Eingabemittels (4) veränderbar und/oder auswählbar ist.

12. Verfahren nach einem der Ansprüche 1 bis 11, **dadurch gekennzeichnet, dass** die unterschiedlichen Regionen (R1, R2, R3) anhand von gemittelten Normalenvektoren (N1, N2, N3) erkannt werden.

13. Verfahren nach einem der Ansprüche 1 bis 12, **dadurch gekennzeichnet, dass** innerhalb einer okklusalen Fläche Höcker und/oder Fissuren als charakteristische Teilbereiche (5) automatisch erkannt werden.

14. Verfahren nach Anspruch 13, **dadurch gekennzeichnet, dass** die Höcker und/oder Fissuren anhand der Krümmung der okklusalen Fläche erkannt werden.

15. Verfahren nach Anspruch 13 oder 14, **dadurch gekennzeichnet, dass** das Tool (T1, T2, T3) auf erkannte Höcker und Fissuren unterschiedlich wirkt.

16. Verfahren nach einem der Ansprüche 1 bis 15, **dadurch gekennzeichnet, dass** das Tool (T1, T2, T3) benachbarte Zähne oder Restaurationen (12) und/oder gegenüberliegende Zähne oder Restaurationen (11) und/oder ein Emergenzprofil (13) aus mindestens einem 3D-Datensatz berücksichtigt.

## Claims

1. Method for computer-assisted processing of a digital 3D model (1) of a dental object by means of digital tools (T1, T2, T3), wherein at least one digital tool (T1, T2, T3) is selected and an effect of the tools (T1, T2, T3) is calculated for the entire 3D model (1), wherein different regions (R1, R2, R3) of the 3D model (1) are identified, wherein at least one region corresponds at least partially to an occlusal or incisal or labial or buccal or distal or mesial or lingual or palatal surface of the dental object,
the tool (T1, T2, T3) has a different effect on different regions (R1, R2, R3),
the calculated effect for the entire 3D model (1) is provided as a proposed model (2) and is displayed together with the unmodified 3D model (1), and
the proposed model (2) is rejected or partially or completely accepted,
wherein, upon complete acceptance, the proposed model (2) is displayed as a result model (6) and, upon partial acceptance, the 3D model (1) as a whole or at least one region (R1, R2, R3) or at least one partial region of the 3D model (1) is selected as the region (10), a result model (6) is produced from the 3D model (1) and the proposed model (2) by replacing the at least one (10) or at least one central part of the selected region (10) with a corresponding region of the proposed model (2) on the basis of the 3D model (1), or approximating it thereto by using at least one strength factor (S), and displaying the result model (6).

2. Method according to Claim 1, **characterized in that** a transition zone is defined around the selected region, wherein, in the transition zone, the 3D model (1) is incrementally aligned with the proposed model (2), which has been weighted by means of the strength factor (S) .

3. Method according to Claim 1 or 2, **characterized in that** the proposed model (2) is transparent or semitransparent in the combined display.

4. Method according to any one of Claims 1 to 3, **characterized in that** regions, in which the proposed model (2) is covered by the 3D model (1) in the combined display, are identified automatically and indicated on the 3D model (1).

5. Method according to any one of Claims 1 to 4, **characterized in that** a distance between the proposed model (2) and the 3D model (1) is indicated with the aid of a color coding of the proposed model (2).

6. Method according to any one of Claims 1 to 5, **characterized in that** the result model (6) and the 3D model (1) are displayed alternately.

7. Method according to any one of Claims 1 to 6, **characterized in that** the at least one selected region (10) is selected by means of an input device.

8. Method according to any one of Claims 1 to 7, **characterized in that**, to select a region (10), a marker (8), which can be moved and selected by means of an input means (4), is displayed on the displayed 3D model (1).

9. Method according to Claim 8, **characterized in that** a region of the proposed model (2) which corresponds to the selected region (10) identified by the marker (8) is displayed in an opaque manner and the selected region (10) of the 3D model (1) is not displayed or is transparent.

10. Method according to any one of Claims 1 to 9, **characterized in that** at least one line (9) is defined and/or changed on the displayed 3D model (1) with the aid of an input means (4).

11. Method according to any one of Claims 1 to 10, **characterized in that** at least one characteristic subregion (5) of the 3D model (1) is identified and displayed automatically and can be changed and/or selected with the aid of an input means (4).

12. Method according to any one of Claims 1 to 11, **characterized in that** the different regions (R1, R2, R3) are identified by means of averaged normal vectors (N1, N2, N3).

13. Method according to any one of Claims 1 to 12, **characterized in that** cusps and/or fissures are automatically identified as characteristic subregions (5) within an occlusal surface.

14. Method according to Claim 13, **characterized in that** the cusps and/or fissures are identified on the basis of the curvature of the occlusal surface.

15. Method according to Claim 13 or 14, **characterized in that** the tool (T1, T2, T3) affects identified cusps and fissures differently.

16. Method according to any one of Claims 1 to 15, **characterized in that** the tool (T1, T2, T3) takes adjacent teeth or restorations (12) and/or opposing teeth or restorations (11) and/or an emergence profile (13) from at least one 3D data set into account.

## Revendications

1. Procédé de traitement assisté par ordinateur d'un modèle 3D numérique (1) d'un objet dentaire, au moyen d'outils numériques (T1, T2, T3), dans lequel au moins un outil numérique (T1, T2, T3) est choisi et un effet de l'outil (T1, T2, T3) pour l'ensemble du modèle 3D (1) est calculé, dans lequel
des régions différentes (R1, R2, R3) du modèle 3D (1) sont reconnues, au moins une région correspondant au moins partiellement à une surface occlusale ou incisive ou labiale ou buccale ou distale ou mésiale ou linguale ou palatine de l'objet dentaire,
l'outil (T1, T2, T3) agit sur différentes régions (R1, R2, R3) de manière différente,
l'effet calculé pour l'ensemble du modèle 3D (1) est mis à disposition en tant que modèle de proposition (2) et affiché conjointement avec le modèle 3D (1) non modifié et en ce que
le modèle de proposition (2) est rejeté ou partiellement ou totalement accepté,
dans lequel, lors de l'acceptation totale, le modèle de proposition (2) est affiché sous forme de modèle de résultat (6) et lors de l'acceptation partielle, le modèle 3D (1) dans son ensemble ou au moins une région (R1, R2, R3) ou au moins une zone partielle du modèle 3D (1) est sélectionné en guise de zone (10), un modèle de résultat (6) est construit à partir du modèle 3D (1) et du modèle de proposition (2), en ce que, en partant du modèle 3D (1), l'au moins une zone sélectionnée (10) ou au moins une partie centrale de la zone sélectionnée (10) est remplacée par une zone correspondante du modèle de proposition (2) ou on s'approche de celle-ci en utilisant au moins un facteur d'intensité (S) et le modèle de résultat (6) est affiché.

2. Procédé selon la revendication 1, **caractérisé en ce qu'**une zone de transition est déterminée autour de la zone sélectionnée, dans la zone de transition, le modèle 3D (1) étant ajusté par étape au modèle de proposition (2) pondéré par le facteur d'intensité (S).

3. Procédé selon la revendication 1 ou 2, **caractérisé en ce que** le modèle de proposition (2) est affiché dans la représentation commune comme transparent ou semi-transparent.

4. Procédé selon une des revendications 1 à 3, **caractérisé en ce que** des zones, dans lesquelles le modèle de proposition (2) est recouvert par le modèle 3D (1) dans la représentation commune, sont reconnues automatiquement et affichées sur le modèle 3D (1).

5. Procédé selon une des revendications 1 à 4, **caractérisé en ce qu'**un écart entre le modèle de proposition (2) et le modèle 3D (1) est affiché au moyen d'un codage couleur du modèle de proposition (2).

6. Procédé selon une des revendications 1 à 5, **caractérisé en ce que** le modèle de résultat (6) et le modèle 3D (1) sont affichés en alternance.

7. Procédé selon une des revendications 1 à 6, **caractérisé en ce que** l'au moins une zone sélectionnée (10) est sélectionnée au moyen d'un appareil de saisie.

8. Procédé selon une des revendications 1 à 7, **caractérisé en ce que**, pour la sélection d'une zone (10), un marquage (8) est indiqué sur le modèle 3D (1) affiché, qui peut être mobile et sélectionné au moyen d'un moyen d'entrée (4).

9. Procédé selon la revendication 8, **caractérisé en ce qu'**une zone correspondant à la zone sélectionnée (10) déterminée par le marquage (8) du modèle de proposition (2) est affichée de manière opaque et la zone sélectionnée (10) du modèle 3D (1) n'est pas affichée ou affichée de manière transparente.

10. Procédé selon une des revendications 1 à 9, **caractérisé en ce que**, par un moyen d'entrée (4), au moins une ligne (9) est définie et/ou modifiée sur le modèle 3D (1) affiché.

11. Procédé selon une des revendications 1 à 10, **caractérisé en ce qu'**au moins une zone partielle caractéristique (5) du modèle 3D (1) est reconnue et affichée automatiquement et modifiable et/ou sélectionnable par un moyen d'entrée (4).

12. Procédé selon une des revendications 1 à 11, **caractérisé en ce que** les différentes régions (R1, R2, R3) sont reconnues par le biais de vecteurs normaux moyens (N1, N2, N3).

13. Procédé selon une des revendications 1 à 12, **caractérisé en ce que**, à l'intérieur d'une surface occlusale, des cuspides et/ou des sillons sont reconnus automatiquement comme zones partielles caractéristiques (5).

14. Procédé selon la revendication 13, **caractérisé en ce que** les cuspides et/ou les sillons sont reconnus au moyen de la courbure de la surface occlusale.

15. Procédé selon la revendication 13 ou 14, **caractérisé en ce que** l'outil (T1, T2, T3) agit différemment sur les cuspides et sillons reconnus.

16. Procédé selon une des revendications 1 à 15, **caractérisé en ce que** l'outil (T1, T2, T3) tient compte, à partir d'au moins un jeu de données en 3D, de dents ou de restaurations (12) voisines et/ou de dents ou de restaurations (11) opposées et/ou d'un profil d'émergence (13).
